# EUROPEAN PATENT SPECIFICATION

(11) **EP 0 712 936 B1**
(45) Date of publication and mention of the grant of the patent: **06.02.2002**
(21) Application number: 94913856.4
(22) Date of filing: 14.04.1994
(51) Int. Cl.: A61K 31/70, A61K 7/00, A61K 7/06, A61K 7/08, A61K 7/50, C07H 21/00, C07H 21/04

(54) **LOW-MOLECULAR DNA FROM STURGEON MILT, A METHOD OF OBTAINING THE DNA AND A PHARMACEUTICAL PREPARATION BASED ON IT**
NIEDERMOLEKULARE DNA AUS STÖRMILCH, METHODE ZUR GEWINNUNG DER DNA UND EIN PHARMAZEUTISCHES PRÄPARAT DARAUF BASIEREND
ADN DE FAIBLE MASSE MOLECULAIRE TIREE DE LAITANCE D'ESTURGEON, PROCEDE D'OBTENTION DE L'ADN ET PREPARATION PHARMACEUTIQUE A BASE DE CELUI-CI

(30) Priority: 10.06.1993 RU 93026472; 11.10.1993 RU 93046326; 11.10.1993 RU 93046327; 11.10.1993 RU 93046328; 11.10.1993 RU 93046329; 19.11.1993 RU 93051600; 24.05.1993 RU 93020154; 24.05.1993 RU 93020155
(43) Date of publication of application: 22.05.1996
(73) Proprietor: NAUCHNO-PROIZVODSTVENNOE PREDPRIYATIE "FARMEK", Moscow, 129226 (RU)
(72) Inventor: ASAFOV, Alexandr Vilenovich, Moscow, 117261 (RU); BEZJULEV, Vyacheslav Vladimirovich, Moscow, 115528 (RU); ANOSOVA, India Grigorievna, Khimki, Moskovskaya obl., 141400 (RU); KONAREV, Alexandr Andreevich, Dolgoprudny, Moskovskaya obl., 141700 (RU); KARPOV, Igor Nikolaevich, Moscow, 125422 (RU); KOCHETKOVA, Marina Gavrilovna, Khimki, Moskovskaya obl., 141400 (RU); TRESCHALIN, Ivan Dmitrievich, Moscow, 115446 (RU); BODYAGIN, Dmitry Alexandrovich, Moscow, 115142 (RU); TRESCHALINA, Elena Mikhailovna, Moscow, 115446 (RU); PEREVERZEVA, Eleonora Rafailovna, Moscow, 119285 (RU); SYRKIN, Anatoly Borisovich, Moscow, 115547 (RU); ILYASHENKO, Viktor Vladimirovich, Moscow, 109542 (RU); SUSULEVA, Natalya Alexandrovna, Moscow, 115446 (RU); DURNOV, Lev Abramovich, Moscow, 113054 (RU); ZOBOVA, Olga Borisovna, Moscow, 103220 (RU); KAZAROVA, Irina Lazarevna, Moscow, 103055 (RU); KOROLEVA, Nelli Borisovna, Moscow, 125047 (RU); KIRAKOSIAN, Stepan Vazgenovich, Moscow, 121158 (RU); SIMONOVA, Lidia Vasilievna, Moscow, 123367 (RU); TARASOVA, Larisa Alexandrovna, Moscow, 125414 (RU); SIMONOVA, Ljudmila Vasilievna, Moscow 125083 (RU)
(74) Representative: Prato, Roberto
(86) International application number: RU9400084
(87) International publication number: WO9428153

(56) References cited:
- EP-A- 0 109 873
- EP-A- 0 360 882
- WO-A-88/06034
- WO-A-94/04551
- US-A- 4 923 978
- US-A- 4 946 952
- US-A- 5 010 183
- US-A- 5 547 684

## Description

The present invention relates to biotechnology, medicine and cosmetology and concerns the low-molecular DNA extracted from milt of sturgeons, a new preparation on its base and a method for extraction of said DNA. The preparation according to the invention possesses a wide range of biological activity and can be utilized in the capacity of a biocorrector, inhibitor of viral activity, HIV (human immunodeficiency virus) infection for one, and lesions of the mucous membrane of various etiology. The preparation will also be useful as a universal cosmetic addition in lotions, creams, dentifrice water, etc.

It is common knowledge that cytostatic therapy, the backbone of medicamentous treatment of tumors, often leads to inhibition of hemopoiesis, even in therapeutic doses (1). In such cases the toxic myelodepression is registered in peripheral blood as leukopenia, thrombocytopenia and, further, as anemia (2). Myelosuppression concomitant with chemo- and chemoradiation therapy limits the treatment possibilities, raises the risk of bacterial infections and lethal termination (1, 2). The range of means used in case of hematogenesis depression is sufficiently wide (3,4). It includes transplantation of bone marrow, introduction of leukomass, colony-stimulating factors, and such preparations as splenin, leukogene, pentoxyl, eimosane and various groups of vitamins (5-7).

However, transplantation of bone marrow is accompanied by additional traumatic intervention while introduction of leukomass may bring about an immune conflict. Pharmaceutic preparations are little effective and call for cancellation of chemotherapy.

In searching for new biologically-active preparations, the leukostimulating effect of nucleic acids has been discovered (8-10). The leukostimulating effect of the preparations based on high-molecular DNA produced from the thymus and spleen of rats was demonstrated in vivo (8,10). However, such preparations exhibit a number of substantial disadvantages, e.g. slow development of leukocytic reaction and absence of stimulation of bone marrow hematogenesis.

The hemocorrector injected in cases of leukopenia and agranulocytosis was sodium salt of ribonucleic acid produced by hydrolysis from yeast (9). In addition to painfulness of injections, the preparation has the same disadvantages, i.e. it stimulates leukopoiesis only, is low effective and calls for cancellation of the course of chemotherapy.

The efficiency was demonstrated of the preparation containing a high-molecular DNA extracted from milt of herrings in combination with daunorubicene in case of leukemia and thrombocytopenia (11).

The sodium salt of low-molecular DNA produced from milt of sturgeons and not used heretofore features unexpected biological activities such as effective stimulation of leukopoiesis, including bone marrow hematogenesis, stimulation of healing the lesions of mucous membranes of various localization and etiologies, inhibition of viral and microbial activities, particularly HIV infection.

Prior to the present invention, the HIV infection was suppressed by nucleotide analogs, e.g. AZT (3-azi-3-desoxythymidine) (12, 13). However, this preparation is effective only at early stages of the disease and, even in therapeutic doses, is highly toxic which leads in clinical use to complications (headache, anemia, disorders of gastroenteric tract) and thus restricts its administration (14,15). Known in the prior art of treating HIV infection is the administration of difluorinated nucleosides (16) which, however, are little effective. The claimed preparation, according to the invention, features a high efficiency with respect to inhibition of HIV infection, and a low toxicity.

Efficiency of DNA was demonstrated on animal models for treatment of experimental gastric ulcer (17).

The preparation based on low-molecular DNA obtained from milt of sturgeons has demonstrated an unexpectedly high efficiency with respect to lesions of oral and pharyngeal mucous membrane of various etiology including those resulting from administration of antibiotics, preparations of heavy metals, and cytostatics.

A method for producing DNA from milt of sturgeons has been disclosed in Inventor's Certificate USSR No. 1410494, 1986, C074221 (04). The method involves homogenization of milt in a standard citrate-salt buffer, separation of cell nuclei by centrifuging, homogenization and deproteinization with a solution of dodecyl sulfate Na at a temperature of 55 C approximately. The cooled mixture is vigorously mixed with kieselguhr (15.5 %) and filtered. The target DNA is precipitated with alcohol in a 1:1 proportion. The high-polymeric DNA is dissolved and subjected to the effect of sound on a magnetostrictor, and filtered, producing 0.6 % DNA in 1/10 standard citrate-salt buffer. Also known is a method for producing DNA from milt of sturgeons by homogenizing milt, washing it with a physiological solution, deproteinizing the DNA-protein complex with protosubtilin Bacillus subtilis (Inventor's Certificate USSR No. 780444, C07H21/04).

Also known in the prior art is a method for production of DNA from sturgeon milt involving homogenizing of milt in a standard citrate-salt buffer (0.87% Na Cl, 0.54 sodium citrate), centrifuging, deproteinizing in presence of dodecyl sulfate Na in the course of 1.5 h at 60 C.

Then the suspension is treated with NaCl and citrate Na to final concentrations of 14.6 % and 24 %, respectively, and heated for 1.5 h at 60 C. To separate proteins, the cooled solution is passed through a column with kieselguhr, producing a DNA solution (Chemo-Pharmaceutical Journal, 1982, No. 7, p. 835-838).

However, all the above-cited methods fail to produce a sodium salt of DNA without polysaccharide admixtures. The presence of even small amounts of polysaccharides in the DNA preparation for medical purposes is undesirable since it brings about a pyrogenic effect. Apart from eliminating this essential disadvantage by producing sodium salt of DNA without admixtures of polysaccharides, the claimed method produces the target substance in the powder form which is quite convenient both for storage and making various pharmaceutic forms of the preparation.

### 1. Method for Extraction of DNA according to the Invention

The present invention relates to a compound of the low-molecular DNA produced from sturgeon milt, to a pharmaceutical preparation containing such a compound, and to a method for extraction of low-molecular DNA from sturgeon milt, as claimed in attached claims 1, 4 and 16 respectively.

In a preferable realization of the process the sturgeon milt is homogenized in a standard citrate-salt buffer solution composed of 0.87 % sodium chloride and 0.54 % sodium citrate. The homogenate is heated at 55-60 C for 1.5 h in presence of sodium dodecyl sulfate (final concentration 96 %). Then the mixture is treated with Na Cl solution to a final concentration of 14.6 % and heated for 1.5 h at 55-60 C. The produced mixture is treated with kieselguhr and filtered. The resulting solution of DNA is passed through an electrodializer at a temperature of 15-20°C and a current density of 1.0-1.5 A/sq dm and/or ultrafiltration plant at a temperature of 20-24°C and a pressure of 2-3 kg/sq cm. The obtained solution is treated by ultrasound, filtered, concentrated if necessary, precipitated with alcohol and the precipitate is washed with 96 % alcohol, dried in a drying cabinet at 40 C, producing DNA in dry powder form.

The salt of DNA obtained by the above-described process features the following properties.

It is a white powder containing at least 80 wt.-% of native sodium salt of low-molecular DNA produced from sturgeon milt, not over 1.5 wt.-% of proteins and not over 17 wt.-% of moisture.

The molecular weight of sodium salt of DNA is not over 12 kD and its hyperchromic effect, not under 37%.

The product prepared in accordance with the claimed method is suitable for medical utilization (market name Polydan).

### 2. Forms of preparation

The preparation according to the invention can be used in different forms for different purposes.

For the purpose of hemocorrection the solution of sodium salt of DNA is used in the form of injections. The diluent may be any pharmaceutically-acceptable diluent including distilled water and physiological solution. The preparation for injection contains DNA in the amount required for exhibiting its hemostimulating effect. The preferable content of DNA in the preparation for injections varies from 1% to 2% since this concentration ensures an optimum pharmacological effect. The claimed preparation is made in factory conditions by a traditional volumetric-weight method (17). A certain weight amount of DNA powder is treated with diluent to the required volume of the solution. Then the solution is filtered, sterilized and poured out into vials. The diluent is preferably distilled water or physiological solution. The resultant solution is a transparent liquid, contains no mechanical or other impurities. It is stable and characterized by long shelf life.

In case of lesions of mouth-and-throat mucous membranes of various etiologies an efficient solution contains DNA in amounts sufficient for healing them. The preferable DNA concentration in the solution is 0.2 to 2.0 %. The diluent may be, apart from physiological solution and water,any other pharmaceutically-acceptable diluent.

For inhibiting HIV infection, DNA is used in combination with polyvalent metals (zinc, nickel, cobalt, iron) in 1:1 to 1:1000 proportions.

The metal systems are produced by mixing components in certain proportions and at different temperatures.

The DNA according to the invention used as a general-purpose biological addition in cosmetics can be utilized in various forms.

The foundation of face-cream contains glycerine stearate, distilled monoglycerides or adequate substitutes thereof, emulsion wax, cosmetic stearin, triethanolamine, glycerine, perfumery oil, conservants, aromatizer, and water.

Hair lotions are based on ethyl alcohol, solubilizer, gelling components, glycerine, 1,2-propyleneglycol, aromatizer, and water.

Lotions are prepared by mixing the gelling components with water then adding the remaining components.

Cream is prepared by a traditional method (18), i.e. by mixing fatty and water phases. DNA is added to the cream mass under certain conditions (temperature, pH, adding sequence).

The basic components of dentifrice water are ethyl alcohol, distilled glycerine, aromatizer, food dyestuffs.

The dentifrice water is prepared by the traditional method (18,19), i.e. by mixing components.

### 3. Studying Toxicity and Mutagenic Activity of the Claimed Preparation

### 3.1. The mutagenic activity was studied in three test systems:

- on indicator bacteria in Ames test (Salmonella) of microsomes, Ames et al, PNAS, 1973, v. 70, p. 2281;
- by registering chromosomal aberrations in bone marrow cells caused by the investigated preparation (Ford C., Stain Technol; 1956, v. 31, p. 247) in a modification (A.M. Malashenko 1977 and G.N. Zolotarev et al, 1978);
- by registering dominant lethal mutations induced by the claimed preparation in embryonal cells of mice (Ehling U.H., Arch. Toxicol., 1978, v. 39, p. 173-185).

In the first test system the concentration of the preparation was 0.1; 1.0; 10; 100 and 1000 mkg/ml.

In the second series of experiments the solution of the studied preparation was prepared directly before administration. The preparation was injected subcutaneously in 0.2 ml of NaCl solution in a dose of 5 mg/kg of animal's body weight (5 toxic doses) and in 100 mg/kg of body weight (100 toxic doses). The control group was injected in the same way with the same volume of diluent. Identical doses were used in the 3rd series of experiments.

An analysis of the obtained results has shown that the studied preparation displays no mutagenic effect in the test range of doses (from subtherapeutic doses to 100 toxic doses).

### 3.2. Studying the Toxicity of DNA Preparation

The preparation in the ready-made form (1.5 % solution) was administered to animals subcutaneously or intravenously one or five times with 24-h intervals depending on the purpose and objects of the experiment. Tests were made on male rats F₁(SVA x C₅₇VC) and SVA, male and female rats Wistar and F (AMSY and Wistar), Guinea pigs, English beagle dogs and chinchilla rabbits. The condition and behavior of animals were observed within a month after the end of the course of administering the preparation.

Within this time, biochemical and clinical analyses of blood, clinical analyses of urine were made and the functional condition of cardiovascular system, etc. was studied.

The toxicity doses were calculated by the Litgfield and Wilkokson method and after a month the animals were killed and subjected to postmortem examination.

The native preparation was injected subcutaneously to male mice F₁ (SVA x C₅₇VC) weighing 18-20 g in doses of 1000, 500 and 250 mg/kg.

Not a single animal perished. Being injected subcutaneously, the preparation in all the above doses exhibited no local irritating effect. The animals endured well the preparation, and the weight gain did not differ from control.

An autopsy discovered no microscopic changes in the organs.

Intravenous injection of the preparation to the mice of the same line was made in the doses of 600, 300 and 150 mg/kg (the volumes were, respectively, 0.3, 0.15 and 0.07 ml per mouse).

The results are summarized in Table 1.

**TABLE 1.**

| Studying Toxicity of DNA Preparation Injected Intravenously to Mice | | |
|---|---|---|
| Dose, mg/kg | Perished animals, % | Time before death, days |
| 600 | 0 | - |
| 300 | 0 | - |
| 150 | 0 | - |

It can be derived from the above table that the dose of 600 mg/kg (equal to 600 therapeutic doses for man) does not kill animals. The weight gain of the surviving animals did not differ from control.

The toxicity of DNA complexes with the above-mentioned polyvalent metals was studied on nonlinear mice weighing 6-7 g by making subcutaneous, intraperitoneal and intracerebral injections in 5, 10, 20, 35 and 50, 75 mg/kg doses. The animals were observed for two weeks after which LD₅₀ was calculated.

The results of experiments have shown that all the tested preparations were nontoxic in the range of doses of 5-75 mg/g regardless of the method of administration.

Investigations were conducted on male mice F₁ (AMSU x Wistar) weighing 220-250 g on an average.

The results appear in table 2.

**TABLE 2.**

| Studying Toxicity of DNA Preparation Administered Subcutaneously to Rats | | | |
|---|---|---|---|
| Group No. | Dose and administration regimen | No. of animals: perished/total | Cumulative dose,mg/kg |
| 1 | 30 mg/kg x 5/24 g | 0/15 | 150 |
| 2 | 6 mg/kg x 5/24 g | 0/15 | 30 |
| 3 | Intact control | 0/15 | |

Other investigations included studying the indices of peripheric blood (clinical and biochemical analyses), taking an electrocardiogram, determining daily diuresis of rats and examining the composition of their urine.

Daily throughout the experiment the body weight of animals was measured.

The pathomorphological examination was conducted by killing 5 animals from each group by decapitation on the 14th and 30th day after the completion of the course. Then the animals were dissected, their internal organs weighed and their weight coefficients determined. Pieces of organs were fixed in a 10-% solution of formalin, colored with hematoxylin-eosine and subjected to optical microscopy.

The examination has shown that the preparation is easily endured by the animals. There were no abnormalities in the behavior and condition of the animals. Administration of the preparation was not accompanied by a pain syndrome.

The animals gained weight normally and the weight coefficients of their organs varied within the limits of the physiological standards.

Examination of the peripheric blood has revealed no essential changes. The number of erithrocytes, hemoglobin, leukocytes and thrombocytes in the test group varied within the limits of physiological norms.

Examinations of the cardiovascular system based on ECG have shown that the preparation exerts no pathological effect on the functioning and electric processes in the cardiac muscle.

Examination of kidney function based on the clinical analysis of urine has revealed that the preparation has no nephrotoxic effect.

The results of investigations lead to a conclusion that the preparation, even in large doses, is nontoxic and well-tolerated by animals.

### 4. Administration of Preparation in the Capacity of a Hemocorrector

### 4.1. Experimental Investigations

The hemocorrecting properties of the preparation have been disclosed on animals (mice, dogs) under the conditions of experimental leukocytopenia caused by cytostatics.

In the first series of experiments the English beagle dogs weighing 11.5-15.6 kg were used. Aplasia of hemogenetic tissue was caused by peroral administration of myelosan in a single dose of 15 mg/kg. The sodium salt of DNA was administered subcutaneously in single doses of 10 mg/kg and 25 mg/kg twice on the 3rd and 7th days after injection of myelosan. The cumulative doses of DNA were, respectively, 20 mg/kg and 50 mg/kg. Dogs in the control group received 10 ml each of physiological solution at the same dates. After 3, 7, 14, 21, 28, 42 and 49 days from the beginning of the experiment the number of leukocytes in peripheric blood and the content of karyocytes of the bone marrow in sternal punctates vere determined.

The results of experiments are summarized in table 3.

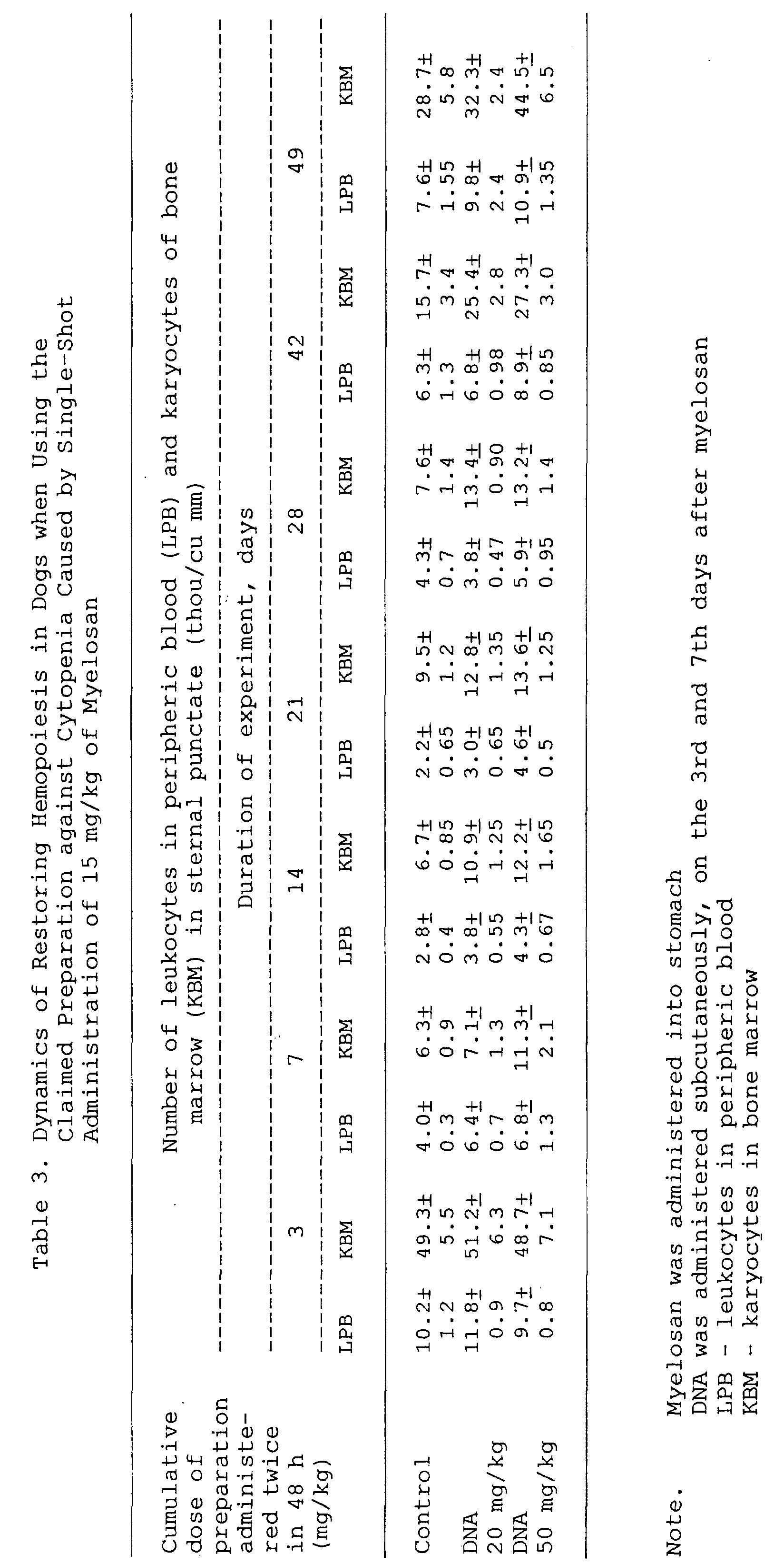

It follows from the tabulated results that administration of myelosan has brought about a radical reduction of leukocyte content in peripheric blood and of karyocytes in the bone marrow.

The content of leukocytes and karyocytes in control animals remained low up to the 28th day of the experiment and began to be slowly restored after the 42th day but failed to reach the initial level to the end of observation, on the 49th day.

Introduction of the sodium salt of DNA brought about an essential change into the picture of toxic action of the cytostatic and favored the faster recovery of the content of bone marrow cells and leukocytes. Thus, after administration of sodium salts of DNA in cumulative doses of 20 and 50 mg/kg leukocytopenia in both groups proved to be not so deep.

The tendency to the recovery of the number of leukocytes in peripheric blood was observed already on the 21st day of the experiment while by the end of observation period the level of leukocytes practically returned to the initial value. A similar picture was observed in the recovery of karyocyte content in bone marrow.

Thus, in this series of experiments on dogs it was shown that the sodium salt of DNA in a cumulative dose of 20 mg/kg and, most conspicuously, in a 50 mg/kg dose contributes to speedier recovery of hematogenetic indices in case of hemopoiesis suppression caused by the cytostatic myelosan.

The second series of experiments was carried out on male hybrid mice F₁ (CBAxS₅₇BS) weighing 18-20 g. Cytopenia was caused by administering cyclophosphane intraperitoneally in single doses of 200 and 275 mg/kg. The sodium salt of DNA was injected subcutaneously by single shots in doses of 150 and 30 mg/kg and on the 3rd, 5th and 7th days after cyclophosphane injection. On the 1st, 3rd, 5th, 7th, 10th, 12th, 14th, 17th, 21st and 25th days the total number of leukocytes in peripheric blood was determined in the Goryaev chamber. The results of the experiment are summarized in Table 4.

It is seen from Table 4 that the maximum cytopenic effect after introduction of cyclophosphane is evolved on the 3rd day while the number of cells is recovered on the 7th-9th day. However, on the 12th-13th day there occurs a second, not so deep reduction of the number of leukocytes.

**TABLE 4**

| Dynamics of Recovery of Hemopoiesis in Mice when Using Claimed Preparation against Cytopenia Caused by Single-Shot Administration of 200 mg/kg of Cyclophosphane | | | | | | | | |
|---|---|---|---|---|---|---|---|---|
| (mk/kg) | Number of leukocytes in peripheric blood (thou/cu mm) | | | | | | | |
| | Duration of experiment, days | | | | | | | |
| | | 3 | 5 | 8 | 10 | 12 | 1 | 17 |
| Control (w/o DNA) | 13.9±3.4 | 2.42±0.27 | 4.46±1.04 | 6.8±2.86 | 8.34±0.77 | 7.58±1.65 | 5.94±0.88 | 6.38±2.31 |
| DNA 30 (single-shot) | 13.1±1.2 | 2.62±0.44 | 8.26±1.8 | 8.86±1.7 | 7.38±1.04 | 8.74±0.55 | 8.02±2.42 | 8.24±4.73 |
| DNA 150 (single-shot) | 12.1±2.5 | 1.9±0.7 | 6.38±0.7 | 9.56±1.2 | 7.82±0.82 | 7.7±1.81 | 7.88±1.37 | 7.52±1.37 |
| DNA 150 (on 3rd; 5th and 7th days) | 11.6±2.36 | 2.44±0.55 | 6.98±1.54 | 10.5±1.92 | 11.64±2.36 | 8.94±2.36 | 7.96±1.43 | 8.86±1.8 |

Administration of 150 mg/kg of DNA on the 3rd, 5th and 7th day achieves a maximum stimulating effect. 2-3 days after the first injection of DNA the number of cells returns to the background level before introduction of myelosan while on the 7th-10th day the number of cells reaches a maximum exceeding said level. It should be noted that, unlike other hemocorrectors, DNA does not cause any compensating reduction of cell number after hemostimulation.

On the whole, the analysis of experimental materials leads to the following conclusions.

As distinct from the known preparation containing yeast RNA, administration of the claimed preparation speeds up the recovery of the leukocyte number in peripheric blood and increases the number of karyocytes in the bone marrow. One injection of DNA preparation is sufficient for producing the stimulating effect while the use of RNA yeast achieves the desired effect only after a 10-days treatment. Unlike the RNA yeast, the claimed preparation does not cause compensating reduction of cell number after hemostimulation.

### 4.2. Clinical Investigations

The clinical investigations of the claimed preparation have been carried out in oncological patients in connection with inhibition of hemopoiesis caused by chemoradiation therapy. The observed group consisted of 30 patients within a 6-14-year age bracket suffering from lymphosarcoma, lymphoblastic leucosis, etc. Treatment with cytostatics and radiation caused the development of leukopenia (100 to 1500 per cu.cm of blood).

The course of treatment included the preferable use of 0.5-2.5-% solution of low-molecular DNA produced from the milt of sturgeons. The doses and number of injections were varied in accordance with the course of the main ailment. The most preferable was the dose interval from 7 to 35 mg/kg ensuring a 3-5-fold increase of leukocytes after 1 or 2 injections.

The results of investigations are presented in Table 5.

**Table 5.**

| Dynamics of Hemogenesis in Oncological Patients Treated by Chemoradiation Therapy with Administration of Polydan | | | | | | |
|---|---|---|---|---|---|---|
| No. of pts | Approximate plan of treatment | Dynamics of leukopenia | | Basic course of chemotherapy | Time for normalization of homogenesis | Compensating reduction of cell number after hemostimulation |
| | | before administration of preparation | after administration of preparation | | | |
| 30 | COPP,CVPP, DOPP and/or pharmorubicine | 100-1500 | 450-5000 | full course | 2-3 days | none |
| | COPP,CVPP, DOPP and radiation therapy | do | do | do | do | do |
| | Pharmo-rubicine and radiotherapy | do | do | do | do | do |
| Note. COPP - cyclophosphane, oncovin, prednisolon, procarbazine CVPP - cyclophosphane, vicblastin, prednisolon, procarbazine DOPP - dopane, oncovin, prednisolon, procarbazine | | | | | | |

An analysis of a myelogram in the observed group of patients has demonstrated not only leukostimulating effect but also a pronounced influence upon normalization of megakaryocytic hemogenesis (Table 6).

**Table 6.**

| Dynamics of Myelogram (%) with Administration of Polydan | | | | |
|---|---|---|---|---|
| No. | Myelogram indices | Before administration | After administration | Norm |
| 1 | Neutrophilic myelocytes | - | 14.0 | 7.0-12.2 |
| 2 | All cells of granulocytic excrescence | 47.5 | 52.5 | 57.1-66.5 |
| 3 | Lymphocytes | 34.0 | 21.0 | 4.3-13.7 |
| 4 | Plasmoblasts | 1.0 | - | - |
| 5 | Polychromatophilic normocytes | 0.5 | 1.0 | 8.9-16.9 |
| 6 | Maturing factor: erythrokaryocytes | - | 0.8 | 0.7-0.9 |
| 7 | Leukoerythroblastic ratio | - | 22.2 | 2.1-4.5 |

Thus, the claimed preparation exhibits not only a leukostimulating effect by far more intensive than that of the previously known preparation but also an ability unknown heretofore to stimulate the bone marrow hemogenesis.

Moreover, unlike the preparations of the prior art level, we have unexpectedly discovered an absence of the phase of compensating reduction of cell quantity after hemostimuation. An important advantage of the preparation according to the invention lies in the possibility for clinical continuation of the main treatment course, i.e there is no necessity for cancelling the chemo-radiotherapy which is a governing factor in treating malignant diseases.

A comparative characteristic of the known preparations used in chemo-radiotherapy and of the claimed preparation appears in Table 7.

**Table 7**

| Comparative Characteristic of Hemostimulating Preparations used in Chemo-radio Myelodepression | | | | | |
|---|---|---|---|---|---|
| Preparation | Administration plan | Effect | | | Complications |
| | | stimulation of leuko poiesis | stimulation of bone marrow hemogenesis | stimulation of thromopoiesis | |
| Granulocyto-macro-phago-colony-stimulating factor GM-KFS | 5 mg/kg per day for 20 days | ++ | ++ | - | Anorexia, fever, vomimiting, leukocytosis, or secondary leukopenia of pancreas and liver |
| Sodium nucleinate (yeast RNA) | 0.5-5 mml of 2-5 % solution 1-2 times a day for 20 days | + | - | - | Painfulness during injection |
| DNA according to invention | single shot, 5 ml of 1.5-% solution | ++ | ++ | + | None |

Sodium nucleinate Na (sodium salt of nucleic acid) produced by hydrolysis of yeast is used as a hemocorrector in cases of leukopenia. The factor of stimulating the colonies of granulocytes and macrophages is the most frequently used hemocorrector in leukopenias resulting from chemo-radiotherapy.

The use of both said preparations involves cancelling the course of main antitumoral therapy.

A comparative characteristic of the leukostimulating effect of the preparation based on high-molecular DNA from the thymus and spleen of rats and of the claimed preparation on animal models appears in Table 8.

**Table 8**

| Comparative Characteristic of Leukopoiesis-Stimulating Preparations (used on animals) | | |
|---|---|---|
| Characteristic of DNA preparation, administration plan | Time for reaching initial level of leukocytes | Effect on bone marrow hemogenesis |
| Produced from thymus and spleen of rats. 2 mg/kg, 4-5 injections (for rats) | 15-25 days | None |
| Sodium salt of DNA produced from sturgeon milt. | 8-21 days | Simultaneous stimulation of bone-marrow hemogenesis |
| Dogs: 20-50 mg/kg, two injections. | | |
| Mice: 30-150 mg/kg, one, three and five injections | | |

The data of experiments and clinical investigations testify to a highly effective hemostimulating effect of the claimed preparation in combination with its nontoxicity and nonmutagenicity which renders it quite suitable for clinical employment as a hemocorrector for a wide range of applications not only in case of cytopenia in oncological patients, caused by chemo-radiotherapy but also in radiological medicine, radiation sicknesses and in other cases associated with deviations of blood formula.

### 5. Administration of the Preparation as a HIV Infection Inhibitor

Prior to the present invention there was no information on the antiviral activity of nucleic acids. The HIV infection can be inhibited, according to the present invention, by the sodium salt of DNA produced from sturgeon milt in combination with polyvalent metals (zinc, nickel, cobalt, iron) in a ratio from 1:1 to 1:1000. The preparation can be administered by subcutaneous, intraperitoneal and intranasal methods or into the cerebrospinal canal, depending on the form of the disease, in therapeutically-equivalent doses. The antiviral effect achieved in different forms of administering the preparation is vital for various clinical forms of AIDS-pulmonary and gastroenteric with damage to the central nervous system. (14). High efficiency of the preparation according to the invention in inhibiting the HIV infection was discovered in the following experiments.

HIV in the cell culture systems was inhibited by the DNA complexes in combination with zinc, cobalt, nickel and iron at various concentrations of components. Azidothymidine manufactured by the Welcome Co. was used for comparison. The antiviral activity of preparations was assessed by the methods recommended for this purpose by the World Health Organization (20, 21). For this purpose we used the cultures of inoculated cell lines SEM and MT-4. The cells were cultured at a concentration of (0.03-0.05)x10⁶ cells per 1 ml of PPM1 1640 with 10-% fetal serum of calves, 300 mg/ml of glutamine, 100 mkg/ml of heptamicine and grown in the form of a suspension. The vital activity of cells was checked by coloring them with a 0.4-% solution of trypan blue. The virus sources were strains HIV/IVS and HIV - 1 HTLV/IIIB.

The suspension of cells was placed into 24-hole boards, treated with different doses of the preparation and infected with HIV. The multiplicity of infection was 0.01 TCD₅₀ per cell. Then the cultures were incubated at 37 C in the atmosphere containing 5% CO₂ at a moisture content of 98% for 5-7 days before determining the cytopathic effect of the virus on the cell culture.

The formation of viral antigen was assessed by immuno-fermental analysis.

The results are given in Table 9.

**Table 9.**

| Efficiency of Inhibiting the HIV infection | | | | | | |
|---|---|---|---|---|---|---|
| Preparation | DNA-metal ratios of components, moles | No of syncytia, % of virus control | Data of immuno-enzyme analysis | | Cytotoxicity | |
| | | | optical density | mg/mg | No of cells per 1 ml of cultural liquid 10 | Vitality of cells, % |
| DNA/ | 1:1 | - | - | - | - | - |
| Zn | 4:9 | - | - | - | - | - |
| | 3:10 | 25 | - | 0.25 | 0.83 | 69 |
| | 0.5:350 | 35 | - | 0.50 | 0.88 | 81.2 |
| | 1:1000 | 70 | 0.487 | - | 0.86 | 84.3 |
| | 1:1100 | 100 | 0.597 | - | 0.89 | 86.2 |
| DNA/ | 1:1 | - | - | - | - | - |
| Ni | 4:9 | - | - | - | - | - |
| | 3:10 | 0 | - | 0.1 | 0.82 | 71.0 |
| | 0.5:350 | 0 | - | over 2.0 | 0.86 | 79.0 |
| | 1:1000 | 68 | - | over 2.0 | 0.85 | 84.3 |
| | 1:1100 | 100 | 0.607 | - | 0.88 | 87.5 |
| DNA/ | 1:1 | - | - | - | - | - |
| Co | 4:9 | - | - | - | - | - |
| | 3:10 | 10 | 0.190 | - | 0.86 | 78.4 |
| | 0.5:350 | 15 | 0.184 | - | 0.84 | 87.1 |
| | 1:1000 | 66 | 0.414 | - | 0.89 | 76.3 |
| | 1:1100 | 100 | 0.585 | - | 0.87 | 82.1 |
| DNA/ | 1:1 | - | - | - | - | - |
| Fe | 4:9 | - | - | - | - | - |
| | 3:10 | 0 | 0.103 | - | 0.87 | 79.3 |
| | 0.5:350 | 0 | 0.247 | - | 0.84 | 82.1 |
| | 1:1000 | 61 | 0.482 | - | 0.87 | 81.7 |
| | 1:1100 | 100 | 0.595 | - | 0.85 | 84.5 |
| AZT (azidothymidine) | | 8 | 0.091 | 0.1 | 0.61 | 55.2 |
| Virus control | | 100 | 0.602 | over 2.0 | - | - |
| Cell control | | 0 | 0.087 | under 0.1 | 0.91 | 86.3 |

It can be seen from the Table that introduction of the DNA-metal complex into the cultural medium is accompanied by inhibition of HIV infection. The optimum DNA-metal relation varies from 1:1 to 1:1000. The virostatic effect is combined with a low cytotoxicity. As distinct from the claimed method, treating the cultured medium with AZT preparation brings about high cytotoxicity with a moderate antiviral effect.

Then toxicity of the preparation according to the invention was investigated on experimental animals.

Tests were made concurrently with all four types of the preparation in comparison with azidothymidine in 5, 10, 20, 35 and 50 mg/kg doses. Investigations of toxicity of the four types of the preparation and azidothymidine were conducted on nonlinear white mice weighing 6-7 g with preparations administered in different doses in the form of subcutaneous, intranasal, intraperitoneal and intracerebral injections. The animals were kept under observation for two weeks and calculations were made by the Curber method. The LD₅₀ was determined as a dose of the preparation causing death of 50% of animals.

The results of toxicity investigations of these preparations are summarized in Table 10.

**Table 10.**

| Toxicity and Anti-HIV Activity of DNA-metal Complexes at Different Modes of Administration | | | | | | | | | |
|---|---|---|---|---|---|---|---|---|---|
| No. | Prepation | Method of adnistration | Dose, mg/kg | | | | | | Qty of syncytia, % |
| | | | 5 | 10 | 20 | 35 | 50 | 75 | |
| 1 | DNA/Zn | intracerebral | 6/6 | 6/6 | 6/6 | 6/6 | 6/6 | 6/6 | 25 |
| | | subcutaneous | 6/6 | 6/6 | 6/6 | 6/6 | 6/6 | 6/6 | 25 |
| | | intraabdominal | 6/6 | 6/6 | 6/6 | 6/6 | 6/6 | 6/6 | 25 |
| | | intranasal | 6/6 | 6/6 | 6/6 | 6/6 | 6/6 | 6/6 | 25 |
| 2 | DNA/Ni | intracerebral | 6/6 | 6/6 | 6/6 | 6/6 | 6/6 | 6/6 | 15 |
| | | subcutaneous | 6/6 | 6/6 | 6/6 | 6/6 | 6/6 | 6/6 | 15 |
| | | intraabdominal | 6/6 | 6/6 | 6/6 | 6/6 | 6/6 | 6/6 | 15 |
| | | intranasal | 6/6 | 6/6 | 6/6 | 6/6 | 6/6 | 6/6 | 15 |
| 3 | DNA/Co | intracerebral | 6/6 | 6/6 | 6/6 | 6/6 | 6/6 | 6/6 | 31 |
| | | subcutaneous | 6/6 | 6/6 | 6/6 | 6/6 | 6/6 | 6/6 | 32 |
| | | intraabdominal | 6/6 | 6/6 | 6/6 | 6/6 | 6/6 | 6/6 | 33 |
| | | intranasal | 6/6 | 6/6 | 6/6 | 6/6 | 6/6 | 6/6 | 31 |
| 4 | DNA/Fe | intracerebral | 6/6 | 6/6 | 6/6 | 6/6 | 6/6 | 6/6 | - |
| | | subcutaneous | 6/6 | 6/6 | 6/6 | 6/6 | 6/6 | 6/6 | - |
| | | intraabdominal | 6/6 | 6/6 | 6/6 | 6/6 | 6/6 | 6/6 | - |
| | | intranasal | 6/6 | 6/6 | 6/6 | 6/6 | 6/6 | 6/6 | - |
| 5 | AZT/(azidothymidine) | intracerebral | 6/6 | 6/6 | 6/6 | 6/6 | 6/6 | 6/6 | 10 |
| | | subcutaneous | 6/6 | 6/6 | 6/6 | 6/6 | 6/6 | 6/6 | - |
| | | intraabdominal | 6/6 | 6/6 | 6/6 | 6/6 | 6/6 | 6/6 | - |
| | | intranasal | 6/6 | 6/6 | 6/6 | 6/6 | 6/6 | 6/6 | 75 |
| 6 | Placebo | intracerebral | 6/6 | 6/6 | 6/6 | 6/6 | 6/6 | 6/6 | 100 |
| | | subcutaneous | 6/6 | 6/6 | 6/6 | 6/6 | 6/6 | 6/6 | 100 |
| | | intraabdominal | 6/6 | 6/6 | 6/6 | 6/6 | 6/6 | 6/6 | 100 |
| | | intranasal | 6/6 | 6/6 | 6/6 | 6/6 | 6/6 | 6/6 | 100 |
| Note. Numerator - quantity of survived mice Denominator - quantity of infected mice. | | | | | | | | | |

One can see in the Table that all the five types of the preparation, regardless of the dose, proved to be nontoxic for the white mice weighing 5-7 g in case of subcutaneous, intranasal and intraabdominal administration (observation term 2 weeks). At the same time 50% of mice perished when the 50 mg/kg dose of DNA was exceeded. Azidothymidine also resulted in mortality of 50% of mice after intracerebral introduction in doses varying from 10 to 50 mg/kg of animals' body weight. It should be noted that the investigated preparation complexes, whatever the method of administration, retain the antiviral activity while in case of AZT it is more conspicuous in case of intracerebral, subcutaneous and intraabdominal injections.

An analysis of experimental data testifies definitely to a pronounced anti-HIV activity of the claimed preparation in combination with its low toxicity regardless of the method of injection.

### 6. The antiviral activity of DNA-metal complexes was also investigated with respect to the influenza virus, adenoviruses and herpes viruses, using the conventional methods (Mc. Canley J., Biochem.J. 1983, v. 224, p. 281; Wigand R. et al, Entevirology, 1982, v. 18, p. 169; Allen G.P., Aust.J. Vet. Res. 1983, v. 44, p. 263). The obtained data are presented in Table 11.

**Table 11**

| Investigation of Viral Activity of DNA-metal Complexes | | |
|---|---|---|
| Type of virus | System used | % of virus growth inhibition (average data) |
| Influenza virus | Culture of chickens' embrios | 21-24 % |
| Adenoviruses | Culture of Hela cells | 25-32 % |
| Herpes viruses | Culture of Hep-Z cells | 45-60 % |

This Table shows that the complexes ensure a sufficient antiviral effect, most conspicuous in herpes virus.

### 7. Stimulating Healing of Lesions of Mucous Membranes

The efficiency of nucleic acids in healing the lesions of mouth and pharynx mucous membranes has been unknown heretofore. The claimed preparation exhibits a possibility of stimulating the healing of lesions of the mouth and pharynx mucous membranes of various etiologies. Most crucial today is the problem of healing the mucous membrane lesions of the oral and pharynx cavity which are frequent complications after local and parenteral administration of antibiotics, preparations of heavy metals, and cytostatics (22-24).

Investigations of DNA according to the invention within a broad range of concentrations (from 0.2 to 2.0%) have demonstrated the efficiency of the preparation in all the studied concentrations with virtually no complications.

The results of clinical investigations of the effect of the DNA sodium salt solution on the dynamics of medicamentous damage to the mucous membrane of the oral and pharyngeal cavity are presented in Table 11. The main diseases of 48 observed oncological patients were lymphosarcoma, histiocytic sarcoma, acute leukosis, lymphogranulomatosis.

The patients were treated with chemotherapeutic preparations, such as methatrexate, vincrysgin, prednisolon, etc.

On the 3rd-5th day of polychemotherapeutic treatment symptoms of stomatitis and gingivitis were observed in the oral and pharyngeal cavity of the patients.

Examination has revealed hyperemia and edemas of the mucous membrane in the oral cavity, numerous elements of aphthosis covered by white patches and acutely painful.

Taking of liquid food was difficult, that of solid food, impossible. Some patients suffered from subfebrile temperature. All patients were given a 5-6-day course of applications of polydan solution 6-8 times a day depending on the degree of the process. The course of polychemotherapy of all the patients was not interrupted.

**Table 12.**

| Dynamics of Clinical Symptoms of Medicamentous Pharyngo-Gingivo-Stomatitis after Applications of DNA solutions | | | | | |
|---|---|---|---|---|---|
| Type of mucous membrane affection | Treatment efficiency, days | | | | |
| | pains vanished | bleeding vanished | hyperemia vanished | ability for taking food | full arresting of process |
| Stomatitises | 2nd day | 2nd-3rd day | 4th-5th day | 2nd-3rd day | 6th day |
| Gingivo-stomatitises | 2nd-3rd day | 2nd-3rd day | 5th day | 3rd day | 7th-8th day |
| Gingivitises | 2nd day | 3rd day | 7th day | 2nd day | 9th day |
| Pharingo-stomatitises | 2nd-3rd day | 3rd-4th day | 8th day | 2nd-3rd day | 10th day |

The Table shows that as early as on the 2nd day of treatment the pains vanished, food could be taken on the 2nd-3rd days and solid food, on the 4th day.

Bleeding of aphtha stopped on the 2nd-4th day and hyperemia, on the 4th-8th day. However, remaining hyperemia here may be associated with revification of granulation processes leading to speedier epithelization of aphthous eruptions.

In all the observed cases the process was fully arrested on the 6th-10th days. And it should be noted that healing of stomatitises occurred on the 6th day, gingivo-stomatitises, on the 7th-8th days, gingivitises, on the 9th day. The longest healing time was registered in case of pharyngeal localization of the process. Here the process was arrested only on the 10th day.

An analysis of the clinical data testifies definitely to the fact that applications of polydan solutions ensure efficient stopping of medicamentous lesions of the mucous membrane in the oral and pharyngeal cavity (stomatitises, gingivitises, gingivistomatitises, pharyingo-stomatitises). An important advantage of this solution lies in that the course of treatment calls neither for cancelling the basic polychemotherapy nor even for reducing the doses.

**Table 13**

| Comparative Characteristic of Effect of Polydan in Combination with Known Preparations in Treating Medicamentous Lesions of the Mucous Membrane of the Oral and Pharyngeal Cavity | | | | |
|---|---|---|---|---|
| Administration | Treatment efficiency (time before full arresting of process) | Possibility of continuing basic treatment | Effect on dynamics of main disease | Condition of mucous membrane of oral cavity |
| Polydan applications acc. to plan | 6-10 days | chemotherapy course continued | ++ | Epithelization, recovery of function |

| Traditional therapy. | | | | |
|---|---|---|---|---|
| Locally: antiseptics and enzymes | 7-14 days | treatment course continued | + | Ulcerous and necrotic process, sometimes of gangrenous nature |
| Orally: antihistaminic and analgetic preparations | 7-14 days | course of chemotherapy cancelled | -- | Epithelization, recovery of function |

It is clearly seen in Table 13 that administration of polydan permits continuing the course of chemotherapy which is beneficial for the dynamics of the main disease. An attempt to continue chemotherapy during traditional treatment has resulted in aggravation of the process on the mucous membrane, even in transition to ulcerous-necrotic and, sometimes, ulcerous-gangrenous forms.

The efficiency of the preparation in treatment of mucous membrane lesions makes for its wide utilization in medicine.

### 8. Cosmetic Effect

The preparation according to the invention exhibits an efficient cosmetic effect. It must be borne in mind that the DNA has not been used heretofore in the capacity of a cosmetic biologically-active addition.

8.1. The efficiency of the claimed preparation in the capacity of a general-purpose biological addition has been demonstrated while studying the curative and prophylactic activity of the cosmetic cream containing an addition of the sodium salt of DNA. These investigations were carried out by the conventional methods (25-26).

Said cream was found to activate the proliferative processes in the skin without any irritating and allergenic effect.

Investigations of the effect of said cream on the biochemical characteristics of the skin have demonstrated that said cream increases the content of total soluble proteins and DNA, and of total lipides. Tests on experimental animals have revealed efficient penetration of DNA marked with C₁₄ thymidine through the skin barrier. The antioxydation, photoprotective and hydrate ability properties have also been discovered. The produced cream is a light-yellowish or white emulsion with a pleasant odor. It is well absorbed and leaves no film on the skin.

The results of the experimental clinical investigations are summarized in Table 14.

**Table 14.**

| Curative and Prophylactic Properties of Cosmetic Cream Containing Sodium Salt of DNA as Biologically-Active Addition | | |
|---|---|---|
| Investigated properties | Investigation methods (information source) | Investigation results |
| Toxicity | (19) | Nontoxic |
| Allergenic effect with morphological control | (26) | Macro- and microscopic examination of skin and internal organs showed no pathological changes |
| Content of total lipides and proteins in skin | Schmidt's method using DNA marked with thymidine C₁₄ (27) | Protein increased from 1.52 to 2.32 DNA increased from 21.0 to 27.4 mg/g Lipides increased from 373.0 to 445 mg/g |
| Photoprotective effect | Spectrophotometry with "Specord" instrument (28) | Cream samples did not pass ultraviolet radiation at wavelength shorter than 280 nm |
| Antioxidating effect | Model of peroxidated lipides (18, 27, 28) | Full inhibition of peroxidation of lipides |
| Hydrate ability | Electrometry with "Teest" system by methods (29) | Pronounced hydrate ability (transcutaneous passage of water) |

These data testify to the fact that introduction of a biologically-active addition - sodium salt of DNA - into the composition of the cosmetic cream has extended the range of its biological activity without any allergenic and other side effects.

8.2. Investigating the curative and prophylactic properties of the hair lotion containing a biologically-active addition in the form of the sodium salt of DNA.

This investigation was carried out in the clinical experiment. The experiments were conducted on puberal animals feeding on the standard ration of a vivarium. Tests were made on 20 male albino Guinea pigs weighing 350-400 g each, and on 5 rabbits, each weighing 2.5-3.0 kg.

Toxicity was investigated by the Gatsuro method (27). The irritating effect was assessed by applying the preparation to the mucous membrane of the rabbit's eye. The tested lotion was also applied daily for 21 days to an area of skin 2x2 cm in size.

The effect of the lotion on the linear growth of hair was studied on Guinea pigs. For this purpose the animal's hair was sheared from a certain area and said area was treated for 21 days with 0.5 g of lotion. On the 7th, 14th, and 21st day the hair was torn out and its length was measured. The structure of hair was studied and the results were compared with the control. Toxicity of the preparation was studied for 21 days. Besides, the general condition, appetite, weight gain, condition of skin, hair and presence of edemas were also observed. Bioptates of skin and internal organs were examined visually and pathomorphologically.

Clinical experiments were carried out on patients of both sexes 14-62 years of age with symptoms of seborrhea and diffuse shedding of hair.

The lotion was rubbed in daily for 21 days. Before the course of treatment, flap samples were made for 24 h under a closed bandage.

Results of experimental clinical investigations: Lotion is nontoxic and exerts no irritating and allergenic effects.

Microscopic analyses have demonstrated regeneration of epiderma cells, an increase in the amount of RNA in the skin, intensification in the derma of the functional activity of histiocytes and mast cells.

A positive influence was established on the structure of hair, strengthening of the hair bursa, matrix and root sheath.

Clinically, the preparation features good organoleptic properties, can easily be applied to the hair-covered part and is devoid of an allergenic effect.

Observation of the seborrhea cases have shown that after a 21-day course the hair was found to be soft, loosely falling with a lower amount of dandruff. The formed film gives efficient protection against weather effects (photo-protective action). Shedding of hair is reduced due to the antioxidating effect. The obtained data are an evidence that introduction of a biologically-active addition - sodium salt of DNA - into the hair lotion produced an effective hair care preparation which reduces the development of seborrhea, has a nutrition effect on hair and exhibits a photoprotective effect.

8.3 Investigating the curing and prophylactic activity of the dentifrice water containing the sodium salt of DNA as a biologically-active addition.

It has been revealed in clinical investigations that the dentifrice water features an antiinflammatory effect, speeds up the healing of the damaged mucous membrane.

Microbiological tests have evolved the antiseptic activity of the dentifrice water.

The clinical tests have evolved excellent taste of the dentifrice water, cleansing effect and a positive influence on parodontium. Thus, the sodium salt of DNA according to the invention imparts an antiinflammatory, antiseptic and antiparodontosis activity to the dentifrice water.

The allergenic tests by conventional methods of all the cosmetic products (26) discovered no allergenic properties.

The obtained results give ground to a conclusion that the sodium salt of DNA is a universal cosmetic addition promoting the curative and prophylactic efficiency of the cosmetic preparation.

Thus, DNA according to the invention is a universal addition to any cosmetic products, features pronounced curative and prophylactic properties without causing any allergenic and other side effects, does not affect adversely the normal structure of the skin and internal organs and is compatible with the active principle of any cosmetic product.

All this offers broad opportunities for extensive use of the salt of DNA according to the invention in the capacity of an addition to cosmetic products.

The invention is illustrated by the following examples.

### EXAMPLE 1

The patient K. admitted to hospital in connection with myelodepression caused by chemo-radiotherapy was administered 5 ml of 1.5 % solution of the sodium salt of DNA.

Before the administration of the preparation, leukopenia was 100-300 leukocytes per cu mm of blood, but after administration a gradual increase in the number of leukocytes was observed. Thus, on the first day after the injection there was 700/cu mm of blood, on the second day, 1000, on the 3rd day, 4200, and on the 5th day, 5100. Another peculiar feature was also positive dynamics of all the granulocytic excrescence cells whose number increased from 47 to 52.5 % (the normal value being 57 %). The maturing index of erythrokaryocytes before treatment with the claimed preparation was equal to zero, while after treatment it was 0.8 (normal value being 0.7-0.9).

There were no observed complications of a general nature, and no irritation or painfulness in the injection area.

### EXAMPLE 2

The patient A., 13, was admitted into the department of infantile oncology on the 13th of July 13, 1992. The diagnosis was acute lymphoblastic leukosis. A course of chemotherapy with cyclophosphane, vinkristin, rubomicine and prednisolon was administered. The result was increasing leukopenia. On November the 25th, 1992 the number of leukocytes was 2500, on November the 27th, 1992, 400, on November the 28th, 1992, 300, and on November the 29th, 100. On November the 30th, 1992 the patient was injected with 5 ml of 1.5% solution of polydan. The result was an increase in the number of leukocytes.

Thus, on December the 2nd, 1992 the number of leukocytes was 700, on December the 7th, 4200, on December the 10th, 5000. Simultaneously, the myelogram was normalized: the number of myelocytes, erythrokaryocytes, polychromatophylic normocytes increased which was an evidence of the stimulation of bone marrow hemogenesis.

As a result of correction of hemopoiesis the course of chemotherapy was not interrupted. During subsequent treatment there was no further reduction in the number of leukocytes. Injection of the polydan solution did not cause any side effects.

### EXAMPLE 3

A culture of inoculated cells, line CEMSS and MT-4 was used in the experiment. The cells were cultivated at a concentration of 0.03x0.05x10⁶ cells per 1 ml of PPMJ 1640 medium with 10-% fetal serum of calves, 300 mg/ml of L glutamin, 100 mkg/ml of hentamycine and were grown in the form of a suspension. The vital activity of the cells was checked by coloring them with a 0.4-% solution of trypan blue. Strains HIV/IVS and HIV-LHTLV/IIIB were used for viral sources. The suspension of cells was placed into 24-hole plates, treated with various doses of DNA-Zn complex at a DNA-metal relation of 1:1, 4:9, 3:10, 0.5:350, 1:1000 and infected with HIV. The multiplicity of infection was 0.01 TCD₅₀ per cell.

Then the cultures were incubated at 37 C in the atmosphere containing 5% of CO at a moisture content of 98% for 5-7 days before determining the cytopathic effect of the virus on the cell culture. The formation of viral antigen was assessed by an immunofermental analysis.

The experiment has shown that the number of syncytia, per cent of virus control at a DNA-metal relation of 3:10 was 25% at 69% of viable cells; at a relation of 0.5:350, 35% at 81.2 % of viable cells; at a relation of 1:1000, 70% and 84.3 % of viable cells; and at a relation of 1:1100, 100% and 86.2 % of viable cells.

### EXAMPLE 4

The experiment was conducted as in Example 3.

The suspension of cells was processed with various doses of DNA-Ni complex at the same relations of components as in Example 1. The experiment has shown that the number of syncytia, per cent of virus control at a DNA:Ni relation of 3:10, 0.5:350 was zero at a viability of cells of 81 and 90%; at a relation of 1:1000, it was 68% at 84.3 % of viable cells and at a relation of 1:1100, it was 100% at 87,5 % of viable cells.

### EXAMPLE 5

The experiment was conducted as in Example 3.

The suspension of cells was treated with various doses of DNA-Co complex at the same relations of components as in Example 1. The experiment has shown that the number of syncytia, per cent of virus control at a DNA-Co relation of 3:10 is 10% at 78.4 % of viable cells; at a relation of 0.5:350 it was 15% at 87.1 % of viable cells; at a relation of 1:1000 it was- 66% at 76.3 % of viable cells, and at a relation of 1:1100 the viability was, respectively, 82.1 %.

### EXAMPLE 6

The experiment was conducted as in Example 1.

The suspension of cells was treated with various doses of DNA-Fe complex at the same relations of components as in Example 1.

The experiment has shown that the number of syncytia, per cent of virus control, at DNA-Fe relations of 3:10 and 0.5:350 was zero at a viability of cells of 79.3 and 82.1 %, respectively. At a DNA-Fe relation of 1:1000 the number of syncytia was 61% at a cell viability of 81.7 %; and at a relation of 1:1100 it was 100% at a cell viability of 84.5 %.

### EXAMPLE 7

The patient P. was admitted with the main diagnosis of sarcoma, IV degree. Was given metatrexate in a dose of 50 ml/sq m. On the fifth day the symptoms of stomatitis developed: the oral cavity turned hyperemic with edemas and aphthous eruptions, sharply painful. Taking of food was impossible. The patient was administered applications of 0.25- % solution of polydan on the first day, eight times with 2-h intervals; on the 2nd-6th days, eight times daily; six times on the 7th day and three times on the 8th day. The dynamics of clinical symptoms of stomatitis was the following: on the 2nd day after treating the mucous membrane the pains vanished, the aphthous symptoms stabilized, bleeding stopped. On the 3rd-4th day the pains vanished, the aphthous elements diminished, the patient started taking food; on the 5th day there were no pains, hyperemia vanished; on the 6th day the process was fully arrested, the mucous membrane of the oral cavity was clean, there were no edemas and hyperemia.

The stomatitis was treated without cancelling chemotherapy.

### EXAMPLE 8

The patient K. was admitted with the main diagnosis of lymphosarcoma, IV degree. Was administered 500 ml/sq m of metatrexate and 2 mg/kg of prednisolon.

On the 4th day after the beginning of polychemotherapy the symptoms of gingivostomatitis developed. Examination has revealed hyperemia and edemas in the oral cavity, bleeding aphthous eruptions on the mucous membrane of the cheeks and gums. Palpation of the mucous membrane and taking of food sharply painful.

The patient was administered applications of 0.5-% solution of polydan: eight times a day (every 2-3 h) on the lst-4th day; six times a day on the 5th-8th days. The dynamics of the clinical symptoms of gingivostomatitis was the following: abatement of pains on the 2nd day after treatment of the mucous membrane; on the 3rd day - vanishing of pains, stopped bleeding, taking of food possible; on the 4th-5th day the hyperemia of the mucous membrane diminished and on the 8th day the process was completely arrested. On the 9th day the mucous membrane of the oral cavity and gums was clean, of pale-pink color, without any signs of edema and hyperemia. The gingivostomatitis was treated without cancelling of polychemotherapy.

### EXAMPLE 9

The patient K. was admitted with the main diagnosis of acute leucosis and treated with 500 mg/sq.m of metatrexate.

On the 4th day after the beginning of chemotherapy the symptoms of esophagostomatitis were observed.

Examination has revealed hyperemia and edemas on the mucous membrane of the oral and pharyngeal cavity, aphthous elements bleeding at palpation. The mucous membrane is sharply painful. Taking of food, particularly solid food is sharply impeded. Applications of 1.5-% solution of polydan were administered on the following program: 8 times (every 2 h) daily on the first 7 days; 6 times on the 8th-9th days; 4 times on the 10th day. The dynamics of medicamentous pharingostomatitis was as follows: on the 3rd day painfulness receded, the patient began taking food, on the 4th day the bleeding and number of aphthae diminished, on the 5th-6th day there remained but a small number of eruptions, on the 7th-9th days hyperemia remained. On the 10th day the process was completely stopped. The mucous membrane of the oral and pharyngeal cavity was found to be clean, of a pink color, free of edemas. Esophagostomatitis was treated without cancelling chemotherapy.

Thus, the above examples confirm the efficiency of the polydan solution for treating medicamentous lesions of the mucous membrane of the mouth and pharynx.

### EXAMPLE 10

Making a cosmetic cream with the general-purpose addition. After weighing out the feedstock components, the fatty and aqueous phases are prepared.

To prepare the aqueous phase, 0.1 (w%) of triethanolamine, 2.0 glycerine and water are heated to 75-80 C. The fatty phase is prepared by heating 0.5 lanolin, 1.0 distilled monoglycerides, 0.5 cosmetic stearine, 2.0 perfumery oil and 1.0 emulsion wax to 85 C.

Next, the fatty and aqueous phases are mixed together. DNA and conservants are added to the cream mass under certain conditions (temperature, pH of medium, adding sequence).

The resulting cream is a homogeneous emulsion of white or light-yellow color with a pleasant odor. The pharmacological investigations of the produced cream have shown that it possesses antioxidating, photoprotective, hydrate, proliferative and antiinflammatory properties. No allergic complications and irritating effect were observed. Cream penetrates efficiently through the skin barrier.

### EXAMPLE 11

Preparing a lotion with an addition of sodium salt of DNA.

2.0 (w%) of gel-forming components is mixed with water and 0.20 glycerine. After the preparation of gel, the following ingredients are introduced in succession: 7.0 ethyl alcohol, 0.2 solubilizer, 0.25 of sodium salt of DNA, and 0.2 of aromatizer.

Lotion is a gel-like liquid with a pleasant odor. Experimental clinical investigations of the produced lotion have shown that it features an antiseborrheal photoprotective and nutrient effect. No side effects and allergenic action was discovered.

### EXAMPLE 12

Preparation of dentifrice water containing sodium salt of DNA as a biologically-active addition.

A container is loaded with 35.0 (w%) ethyl alcohol, 5.0 glycerine, distilled water, 0.25 DNA, 0.005 food dyestuffs (indigocarmine blue and yellow tartrasine) and 1.0 of aromatizer. All ingredients are mixed.

The resulting dentifrice water is a bluish liquid with pleasant odor and taste. Clinical investigations have disclosed its antiinflammatory, wound-healing and antiseptic effect without any allergic and other complications.

Thus, the above examples illustrate the favorable properties of DNA used as a universal cosmetic addition, general-purpose cosmetic addition.

### EXAMPLE 13

After weighing out the feedstock components, the fatty and aqueous phases are prepared. To prepare the aqueous phase, 3.0 (w%) glycerine or 1.2 propylene and 0.15 triethanolamine are heated to 75-80 C. Then the fatty phase is prepared by melting to 80-85 C 1.0 anhydrous lanolin, 1.0 distilled monoglycerides or glycerine monostearate or DEG stearate, 2.0 cosmetic stearine, 2.0 perfumery oil or butylstearate or isopropylpalmitate or propylmiristate, 1.0 bee wax, 2.0 of mixture of mono- and diesters, polyethylene glycol-400 and stearate. Then the fatty and aqueous phases are mixed and doped with 3.33 conservants, 0.25 sodium salt of DNA, and 0.4 aromatizer.

The resulting cream is a homogeneous thick white mass with a pleasant smell. The pharmacological investigations of the obtained cream have exhibited the presence of hydrate ability, photoprotective, antioxidating and antiinflammatory properties.

No allergic complications were observed. The cream was found to penetrate readily through the skin barrier.

### EXAMPLE 14

The feedstock components are weighed out then the aqueous phase is made by taking 3.0 (w%) glycerine or 1.2 propyleneglycol, 0.2 copolymer of acrylic acid and polyallyl ethers of pentaerythritol or sodium salt of copolymer of acrylic acid and heating them to 75-80 C. The fatty phase is made by taking 0.5 oxyethylated lanolin, 1.0 distilled monoglycerides or glycerine monostearate or stearate DEG, 1.0 bee wax, 3.0 perfumery oil or butylstearate or isopropylpalminate, 3.0 of a mixture of mono- and diesters of polyethyleneglycol-400 and stearine, 2.0 of a mixture of mono- and diesters of oleic acid and polyethyleneglycol-400, 0.5 of alcohols, fraction C₁₇-C₁₈ and heating them to 80-85 C. Then the aqueous and fatty phases are mixed and doped with 3.33 complex conservant, 0.25 sodium salt of DNA and 0.2 aromatizer. The resulting cream is a homogeneous white or light-yellow emulsion with a pleasant odor. The pharmacological investigations of the produced cream have revealed the presence of its antioxidating, photoprotective, hydrating, proliferating and antiinflammatory properties. There were no allergic complications and irritating effect. The 'cream penetrates efficiently through the skin barrier.

### REFERENCES

1. S.M. Slinchak, Oncology, Kiev "Vysshaya Shkola", 1982, p. 199-204.
2. M.L. Gershanovich. Complications in Chemo-Hormonotherapy of Malignant Tumors. Moscow, "Meditsina", 1982, p. 98-142.
3. M.D. Mashkovsky, Medicinal Drugs. Moscow, "Meditsina", 1985, p. 172, vol. 2.
4. Register of Medicinal Drugs of Russia. Moscow, "Interfarmkhim", 1933, p. 603.
5. M.Bregni S. Siena Breakthrough in cytokine therapy; an overview of GM-CSF. Royal Society of Medicine Services Int. Congress and Symposium Series No. 170, p. 491-6.
6. P. Gupta et al. Bone Marrow Transplantation, 1992, No. 9, p. 491-492.
7. P. Riikonen, U. Saarinen, Medical and Pediatric Oncology, 1992, No. 20, p. 489-496.
8. A.M. Belous. Exogenous Nucleic Acids and Reducing Processes. Moscow, "Meditsina", 1974, p. 126-140.
9. V.V. Rychnev. Nucleic Acids and Their Therapeutic Use. "Vrachebnoye Delo", 1981, No. 8. p. 114-118.
10. E.P. Soboleva. Administratioin of DNA in Cytopenia Caused by Myelosan. Bul. of exper. biol. and med., 1976, vol. 81, No. 4, p. 409-411.
11. G. Cahrton, DNA as a carrier of outra cyclines in the treatment of acute myelocytic leukemia. Hematology, 1991, v. 76, No. 4, p. 99.
12. I.E. Lalayants, L.S. Milovanova. Pharmacological Aspects of AIDS. "Pharmacology and Toxicology. 1989, vol. 52, No. 3, p. 101-110.
13. IPC-5 A 61 K 3170, France, Application No.2643558, publ. 31.08.90.
14. R.V. Petrov, A.M. Borisova. Modern Problems of Diagnosis, Treatment and Prophylaxis of AIDS. "Immunology", 1987, No. 3, p. 5-10.
15. B. Franza B. Characterization of cellular proteins recognizing the HIV enhancer using a microscate DNA-affinity precipitation assay, Nature, 1987, v. 330, p. 391-395.
16. IPC-4 A61 9/66, 37/22, PCT (WO) No. 89/02733, publ. 6.4.89.
17. A.S. Loginov et al. Reparative Effect of Nucleic Acid Preparations in Experimental Gastric Ulcer. Bul. exp. biol. and med., 1991, No. 7, p. 59-60.
18. I.I. Wolfensohn, Cosmetics Today, "Khimia", 1988, p. 11-49.
19. Medical Cosmetics (Manual), ed. N.Mikhailova, "Meditsina", 1984, p. 31-44.
20. Animal Models for Studying HIV and AIDS: memorandum of conference of the World Health Organization, Bul. of WHO 1988, v. 66, No. 5, p. 22-36.
21. Acquired Immunodeficiency Syndrome (AIDS): Conclusions and Recommendations. Bulletin of WHO, 1985, v. 63, No. 4, p. 18-19.
22. N.N. Bazhanov, Stomatology. Moscow, 1984.
23. E.V. Borovsky et al. Therapeutic Stomatology. Moscow, 1973, p. 318-323.
24. Medicamentous Disease. Ed. Mazhdrakova, Sofia, 1973, p. 113-124.
25. N.B. Koroleva. Principle of Experimental and Clinical Assessment of Cosmetic Products. Theses of All-Union Congress of Dermatologists. Moscow, 1979.
26. N.M. Berezhnaya. Allergology (Reference Book), Kiev, "Naukova Dumka", 1986, p. 291.
27. N.B. Koroleva. Principles of Experimental and Clinical Assessment of Cosmetic Products. Theses of All-Union Congress of Dermatologists, Moscow, 1979.
28. N.B. Koroleva. Scientific Approach to the Use of Some Biologically-Active Substances in Cosmetology. Bul. of Dermatology and Venerology, 1977, No. 6, p. 8-12.
29. A.M. Sudarev. Electrometric Method for Investigating Parameters of Hydratant Properties and Microcirculation of Skin. Thesis for the Candidate's Degree. Moscow, 1990.
30. N.B. Koroleva et al. Experimental Clinical Investigation of Biochemical Mechanisms of Skin Aging and their Pharmacological Correction. Transactions of All-Union Congress of Dermatologists. Moscow, 1979.

## Claims

1. A compound of the low-molecular DNA produced from sturgeon milt, containing at least 80 wt.-% sodium salt of DNA, not over 1.5 wt.-% proteins, and not over 17 wt.-% moisture; said sodium salt of DNA being substantially free from polysaccharides, having molecular mass not over 12.0 MD and having a hyperchromatic effect not under 37%.

2. The compound according to Claim 1 **CHARACTERIZED in that** said DNA is combined with polyvalent metals.

3. The compound according to Claim 2 **CHARACTERIZED in that** the metals are selected from the group including Zn, Co, Ni, Fe, Pd, Pt.

4. A pharmaceutical preparation containing an effective amount of active principle in the form of low-molecular DNA of sturgeon milt according to Claim 1 in combination with a pharmaceutically-acceptable additive, diluents or fillers.

5. The pharmaceutical preparation according to claim 4 **CHARACTERIZED in that** said low-molecular DNA of sturgeon milt is in combination with polyvalent metals.

6. The pharmaceutical preparation- according to Claim 4 or 5 **CHARACTERIZED in that** it has the form of a solution.

7. The pharmaceutical preparation according to Claim 6 **CHARACTERIZED in that** it is administrable in the form of injections.

8. The pharmaceutical preparation according to Claim 6 **CHARACTERIZED in that** it is intended for external use.

9. The pharmaceutical preparation according to Claim 7 **CHARACTERIZED in that** its preferable DNA content is from 0.5 to 2.5 %.

10. The pharmaceutical preparation according to Claim 8 **CHARACTERIZED in that** its preferable content of said DNA is from 0.2 to 2 %.

11. The pharmaceutical preparation according to Claim 5 **CHARACTERIZED in that** the relation of DNA and polyvalent metal is from 1:1 to 1:1000.

12. The compound according to Claim 1 **CHARACTERIZED in that** it is used in the form of a cosmetic product in combination with pharmaceutically-acceptable carriers, fillers, additives.

13. The compound according to Claim 12 **CHARACTERIZED in that** it is a dentifrice water or tooth paste.

14. The compound according to Claim 12 **CHARACTERIZED in that** it is a lotion.

15. The compound according to Claim 12 **CHARACTERIZED in that** it has the form of a cream or gel.

16. A method for extraction of low-molecular DNA from sturgeon milt according to claim 1 involving homogenization of frozen milt in a citrate-salt buffer, deproteinizing DNA by detergents, denaturating and separating protein, subsequent degradation of DNA, separating it from specific admixtures and separating the target product **CHARACTERIZED in that** degradation of DNA is preceded by the electrodialysis and/or ultrafiltration stages and the target product is precipitated in the form of a powder from salt solutions with ethyl alcohol.

17. The method according to Claim 16 **CHARACTERIZED in that** ultrafiltrating concentration is carried out at a temperature of 20-24°C and a pressure of 2-3 kg/sq cm.

18. The method according to Claim 16 **CHARACTERIZED in that** electrodialysis is carried out at a temperature of 15-20°C and a current density of 1.0-1.5 A/sq dm.

## Patentansprüche

1. Verbindung der niedermolekularen DNA, welche aus Störmilch hergestellt ist, mindestens 80 Gew,-% eines Natriumsalz von DNA, nicht mehr als 1,5 Gew.-% an Proteinen und nicht mehr als 17 Gew.-% an Feuchtigkeit enthält, wobei das Natriumsalz von DNA ohne eine wesentliche Menge an Polysacchariden ist, ein Molekulargewicht von nicht mehr als 12,0 MD besitzt und einen hyperchromen Effekt von nicht weniger als 37% besitzt.

2. Verbindung nach Anspruch 1, **dadurch gekennzeichnet, daß** die DNA mit mehrwertigen Metallen kombiniert ist.

3. Verbindung nach Anspruch 2, **dadurch gekennzeichnet, daß** die Metalle aus der Zn, Co, Ni, Fe, Pd, Pt einschließenden Gruppe ausgewählt sind.

4. Pharmazeutisches Präparat, welches eine wirksame Menge eines aktiven Grundbestandteils in der Form von niedermolekularer DNA aus Störmilch gemäß Anspruch 1 in Kombination mit einem pharmazeutisch akzeptablen Additiv, Verdünnungsmitteln oder Füllstoffen enthält.

5. Pharmazeutisches Präparat nach Anspruch 4, **dadurch gekennzeichnet, daß** die niedermolekulare DNA aus Störmilch mit mehrwertigen Metallen kombiniert ist.

6. Pharmazeutisches Präparat nach Anspruch 4 oder 5, **dadurch gekennzeichnet, daß** es die Form einer Lösung besitzt.

7. Pharmazeutisches Präparat nach Anspruch 6, **dadurch gekennzeichnet, daß** es in der Form von Injektionen verabreichbar ist.

8. Pharmazeutisches Präparat nach Anspruch 6, **dadurch gekennzeichnet, daß** es für eine äußere Anwendung gedacht ist.

9. Pharmazeutisches Präparat nach Anspruch 7, **dadurch gekennzeichnet, daß** dessen bevorzugter DNA-Gehalt von 0,5 bis 2,5% reicht.

10. Pharmazeutisches Präparat nach Anspruch 8, **dadurch gekennzeichnet, daß** dessen bevorzugter Gehalt der DNA von 0,2 bis 2% reicht.

11. Pharmazeutisches Präparat nach Anspruch 5, **dadurch gekennzeichnet, daß** das Verhältnis von DNA und mehrwertigem Metall von 1:1 bis 1:1000 reicht.

12. Verbindung nach Anspruch 1, **dadurch gekennzeichnet, daß** sie in der Form eines kosmetischen Produkts in Kombination mit pharmazeutisch akzeptablen Trägern, Füllstoffen, Additiven verwendet wird.

13. Verbindung nach Anspruch 12, **dadurch gekennzeichnet, daß** sie ein Zahnputzwasser oder eine Zahnpasta ist.

14. Verbindung nach Anspruch 12, **dadurch gekennzeichnet, daß** sie eine Lotion ist.

15. Verbindung nach Anspruch 12, **dadurch gekennzeichnet, daß** sie die Form einer Creme oder eines Gels besitzt.

16. Verfahren zur Extraktion von niedermolekularer DNA aus Störmilch gemäß Anspruch 1, welches beinhaltet ein Homogenisieren von gefrorener Milch in einem Citratsalzpuffer, Deproteinisieren von DNA mittels Detergentien, Denaturieren und Separieren von Protein, anschließendes Abbauen von DNA, dessen Separieren von spezifischen Zusätzen und Separieren des Zielproduktes, **dadurch gekennzeichnet, daß** diesem Abbau von DNA die Elektrodialyse und/oder Ultrafiltrationsstufen vorangehen und das Zielprodukt mit Ethylalkohol in der Form eines Pulvers aus Salzlösungen ausgefällt wird.

17. Verfahren nach Anspruch 16, **dadurch gekennzeichnet, daß** eine Ultrafiltrationskonzentrierung bei einer Temperatur von 20-24°C und einem Druck von 2-3 kg/cm² durchgeführt wird.

18. Verfahren nach Anspruch 16, **dadurch gekennzeichnet, daß** eine Elektrodialyse bei einer Temperatur von 15-20°C und einer Stromdichte von 1,0-1,5 A/dm² durchgeführt wird.

## Revendications

1. Composé de l'ADN de faible poids moléculaire produit à partir de laitance d'esturgeon, contenant au moins 80 % en poids de sel sodique d'ADN, pas plus de 1,5 % en poids de protéines et pas plus de 17 % en poids d'humidité ; ledit sel sodique d'ADN étant exempt d'une quantité substantielle de polysaccharides, présentant une masse moléculaire inférieure ou égale à 12,0 MD et ayant un effet hyperchromatique au moins égal à 37 %.

2. Composé selon la revendication 1, **caractérisé en ce que** ledit ADN est combiné avec des métaux polyvalents.

3. Composé selon la revendication 2, **caractérisé en ce que** les métaux sont sélectionnés dans le groupe constitué de Zn, Co, Ni, Fe, Pd et Pt.

4. Préparation pharmaceutique contenant une quantité efficace de principe actif sous la forme d'ADN de faible poids moléculaire de laitance d'esturgeon selon la revendication 1, en combinaison avec un additif, des diluants ou des matières de charge acceptables d'un point de vue pharmaceutique.

5. Préparation pharmaceutique selon la revendication 4, **caractérisée en ce que** ledit ADN de faible poids moléculaire de laitance d'esturgeon se trouve en combinaison avec des métaux polyvalents.

6. Préparation pharmaceutique selon la revendication 4 ou 5, **caractérisée en ce qu'**elle présente la forme d'une solution.

7. Préparation pharmaceutique selon la revendication 6, **caractérisée en ce qu'**elle peut être administrée sous la forme d'injections.

8. Préparation pharmaceutique selon la revendication 6, **caractérisée en ce qu'**elle est destinée à un usage externe.

9. Préparation pharmaceutique selon la revendication 7, **caractérisée en ce que** sa teneur préférable en ADN est comprise entre 0,5 et 2,5 %.

10. Préparation pharmaceutique selon la revendication 8, **caractérisée en ce que** sa teneur préférable en dit ADN est comprise entre 0,2 et 2 %.

11. Préparation pharmaceutique selon la revendication 5, **caractérisée en ce que** le rapport entre ADN et métal polyvalent est compris entre 1:1 et 1:1 000.

12. Composé selon la revendication 1, **caractérisé en ce qu'**il est utilisé sous la forme d'un produit cosmétique en combinaison avec des agents véhiculeurs, des matières de charge et des additifs acceptables d'un point de vue pharmaceutique.

13. Composé selon la revendication 12, **caractérisé en ce qu'**il s'agit d'une eau dentifrice ou d'une pâte dentifrice.

14. Composé selon la revendication 12, **caractérisé en ce qu'**il s'agit d'une lotion.

15. Composé selon la revendication 12, **caractérisé en ce qu'**il présente la forme d'une crème ou d'un gel.

16. Procédé d'extraction d'ADN de faible poids moléculaire à partir de laitance d'esturgeon selon la revendication 1, impliquant l'homogénéisation de laitance congelée dans un tampon de sel citrate, la déprotéinisation de l'ADN par des détersifs, la dénaturation et la séparation de la protéine, la dégradation ultérieure de l'ADN, la séparation de celui-ci de mélanges spécifiques et la séparation du produit cible, **caractérisé en ce que** la dégradation de l'ADN est précédée par les étapes d'électrodialyse et/ou d'ultrafiltration et **en ce que** le produit cible est précipité sous la forme d'une poudre à partir de solutions salines avec de l'alcool d'éthyle.

17. Procédé selon la revendication 16, **caractérisé en ce que** la concentration par ultrafiltration est réalisée à une température comprise entre 20 et 24 °C et une pression comprise entre 2 et 3 kg/cm².

18. Procédé selon la revendication 16, **caractérisé en ce que** l'électrodialyse est réalisée à une température comprise entre 15 et 20 °C et une densité de courant comprise entre 1,0 et 1,5 A/dm².
